# EUROPÄISCHE PATENTSCHRIFT

(11) **EP 0 601 224 B1**
(45) Veröffentlichungstag und Bekanntmachung des Hinweises auf die Patenterteilung: **15.10.1997**
(21) Anmeldenummer: 92120836.9
(22) Anmeldetag: 07.12.1992
(51) Int. Cl.: A61F 2/34

(54) **Verankerungsschale zur Aufnahme eines Pfannenkörpers einer künstlichen Hüftgelenkspfanne**
Fixation shell for receiving the cup body of an artificial acetabular cup
Coquille de fixation pour recevoir la coupole d'une coupole de hanche synthétique

(43) Veröffentlichungstag der Anmeldung: 15.06.1994
(73) Patentinhaber: PLUS ENDOPROTHETIK AG, 6343 Rotkreuz (CH)
(72) Erfinder: Zweymüller, K., Prof. Dr. med., A-1180 Wien (AT); Deckner, André, F-75015 Paris (FR)
(74) Vertreter: Popp, Eugen, Dr.

(56) Entgegenhaltungen:
- EP-A- 0 120 595
- EP-A- 0 296 274
- EP-A- 0 341 199
- EP-A- 0 346 270
- EP-A- 0 495 341
- FR-A- 2 676 172
- US-A- 4 662 891

## Beschreibung

Die Erfindung betrifft eine Verankerungsschale aus Metall zur Aufnahme eines Pfannenkörpers aus Kunststoff, einer künstlichen, im Beckenknochen zementfrei verankerbaren Hüftgelenkspfanne gemäß dem Oberbegriff des Anspruches 1.

Eine derartige Verankerungsschale ist aus der EP-A-0296274 bekannt. Diese Verankerungsschale ist aus einem dem Beckenknochen unmittelbar zugewandten, stirnseitigen Bereich und einer sich an den stirnseitigen Bereich anschließenden, konisch zulaufenden Wand gebildet. Dabei ist im stirnseitigen Bereich eine zentrale, mit einem Gewinde versehene Bohrung zur Aufnahme eines Einsetzinstrumentes dieser Verankerungsschale bei der Operation angeordnet. Da die Längsachse des in die Gewindebohrung einschraubbaren Einsetzinstrumentes mit der Längs- bzw. Rotationsachse dieser Verankerungsschale zusammenfällt, wird die Ausrichtung dieser Verankerungsschale durch Richtungsänderung der Achse des Einsetzinstrumentes erheblich erleichtert. Des weiteren weist diese Verankerungsschale im stirnseitigen Bereich mindestens eine Öffnung auf, um ein Einbringen und Verdichten von spongiösem Gewebe, insbesondere von autologem oder homologem Knochenmaterial, am "Boden" der operativ geschaffenen, die künstliche Hüftgelenkspfanne aufnehmenden Ausnehmung im Beckenknochen und ein Hinterfüllen dieser Verankerungsschale mit solchem Material zu ermöglichen. Schließlich ist diese Verankerungsschale noch mit einem selbstschneidenden Gewinde, das auf der Außenfläche der sich an den stirnseitigen Bereich anschließenden, konisch zulaufenden Wand angeordnet ist, ausgestattet. Auf diese Weise kann diese Verankerungsschale ausgesprochen einfach und lagegenau in den Beckenknochen eingebracht und gleichzeitig fixiert werden.

Wie oben erwähnt, dient die Öffnung im stirnseitigen Bereich der Verankerungsschale zum Einbringen und Verdichten von spongiösem Gewebe, insbesondere von autologem oder homologem Knochenmaterial, um den Hohlraum zwischen der Verankerungsschale und dem Beckenknochen zu hinterfüllen. Auch ermöglicht diese Öffnung eine optische Kontrollmöglichkeit von Seiten des Operateurs während des Implantationsvorgangs. Es läßt sich beobachten, ob die Verankerungsschale parallel zum "Boden" der in den Beckenknochen vorgefrästen Ausnehmung eingebracht bzw. eingedreht wird. Des weiteren lassen sich die Eindringtiefe und der Zeitpunkt, zu welchem der stirnseitige Bereich der Verankerungsschale an dem "Boden" der in den Beckenknochen vorgesehenen Ausnehmung zur Anlage kommt, genau feststellen. Damit einhergehend ist eine rechtzeitige Beendigung des Eindringens bzw. Eindrehens der Verankerungsschale in die Ausnehmung sichergestellt. Insofern ist auch einem möglichen Ausreißen des selbstschneidenden Gewindes an der Außenfläche der Verankerungsschale entgegengewirkt. Die erwähnte Öffnung im stirnseitigen Bereich der Verankerungsschale wird beim Stand der Technik durch den aus Kunststoff bestehenden Pfannenkörper bzw. das aus Kunststoff bestehende Inlay verschlossen. Dies bedeutet, daß der Knochen mit dem aus Kunststoff bestehenden Pfannenkörper in Berührung kommt. Es hat sich gezeigt, daß dieser Kontakt für den Heilungsprozeß nachteilig ist und zu Langzeitschäden an dem die Verankerungsschale umgebenden Knochen führen kann. Des weiteren ist beim Stand der Technik nicht ausgeschlossen, daß durch die Öffnung im stirnseitigen Bereich der Verankerungsschale hindurch eingebrachtes Knochenmehl, Knochenspäne, Knochenpartikel oder dergleichen während der Operation wieder herausfallen. Die Folge ist, daß sich dann zwischen Verankerungsschale und Pfannenkörper Knochenpartikel ansammeln, die einem festen Sitz des Pfannenkörpers in der Verankerungsschale entgegenstehen.

Der vorliegenden Erfindung liegt daher die Aufgabe zugrunde, eine Verankerungsschale der aus der EP-A-0 296 274 bekannten Art zu schaffen, bei der die vorerwähnten Nachteile in bezug auf die Öffnung im stirnseitigen Bereich der Verankerungsschale vermieden sind, bei der also insbesondere ein unmittelbarer Kontakt zwischen Knochen und dem aus Kunsstoff bestehenden Pfannenkörper vermieden wird. Diese Aufgabe wird durch die kennzeichnenden Merkmale des Anspruches 1 gelöst. Die erfindungsgemäß ausgebildete Verankerungsschale erlaubt eine Spongiosaplastik in herkömmlicher Weise. Durch die Möglichkeit, daß die im stirnseitigen Bereich der Verankerungsschale vorgesehene Öffnung verschließbar ist, kann vermieden werden, daß hinterfülltes Knochenmaterial während der Operation in den Aufnahmeraum der Verankerungsschale zurückfällt. Des weiteren kann dadurch vermieden werden, daß Knochenmaterial unmittelbar in Kontakt kommt mit dem aus Kunststoff bestehenden Pfannenkörper. Dadurch läßt sich der Heilungsprozeß nach heutigen Erkenntnissen erheblich beschleunigen. Auch lassen sich auf diese Weise Langzeitschäden an dem die Verankerungsschale umgebenden Knochen vermeiden.

Vorteilhafte konstruktive Details der erfindungsgemäß ausgebildeten Verankerungsschale sowie konstruktive Weiterentwicklungen derselben sind in den Unteransprüchen beschrieben. Dabei sei darauf hingewiesen, daß es aus der US-A-4,662,891 bereits an sich bekannt ist, den Umfangsrand einer Verankerungsschale außenseitig als Doppelkonus auszubilden. Durch diese Maßnahme läßt sich ein großflächiger und damit entsprechend fester Kontakt zwischen Verankerungsschale und Knochen herstellen. Des weiteren weist durch die Ausgestaltung der Verankerungsschale gemäß Anspruch 2 diese in operationstechnischer Hinsicht den Vorteil auf, daß eine Verkantung der Verankerungsschale bei deren Einbringen bzw. Eindrehen in die vorgefräste Ausnehmung des Beckenknochens aufgrund einer verbesserten Selbstzentrierung sicher vermieden wird. Auch erhält der stirnseitige Bereich der Verankerungsschale infolge einer weiteren konisch zulaufenden Wand eine zusätzliche Biegesteifigkeit. Schließlich ist die Form der Verankerungsschale gemäß Anspruch 2 anatomisch besser an die sphärische Form des Acetabulums angepaßt. Dies ist für eine hohe Kippstabilität einerseits und große Vorspannung in der knöchernen Hüftgelenkspfanne andererseits besonders vorteilhaft, wodurch eine schnelle Osteointegration des Implantates und ein verbesserter Schutz gegen Migration aufgrund vorhandener Kippbewegungen und sonstiger Widerstände gegeben sind. Ungeachtet dessen ist aufgrund einer derartigen doppelkonischen Ausgestaltung der erfindungsgemäßen Verankerungsschale nur mehr eine geringe Knochenresektion im Bereich des "Bodens" der Hüftgelenkspfanne notwendig.

Vorzugsweise werden für die konisch zulaufenden Umfangswandabschnitte Winkel entsprechend den Ansprüchen 3 und 4 gewählt.

Von ganz besonderer Bedeutung für eine gute Zugänglichkeit wie auch eine hohe Biegesteifigkeit sind entsprechend den Maßnahmen nach Anspruch 5 drei etwa gleich große Öffnungen vorgesehen, die über den Umfang des stirnseitigen Bereiches gleichmäßig verteilt sind. Auf diese Weise werden die über die zentrale Gewindebohrung zur Aufnahme des Einsetzinstrumentes auf den stirnseitigen Bereich übertragenen Kräfte - während des Einbringens bzw. Eindrehens der Verankerungsschale in die dafür vorgesehene Ausnehmung des Beckenknochens - über drei im wesentlichen identisch ausgebildete Verbindungsstücke oder dergleichen auf die beiden etwa konisch zulaufenden Wände hin zu dem selbstschneidenden Gewinde zu gleichen Teilen abgeleitet. Einer Verformung des stirnseitigen Bereiches der Verankerungsschale ist somit entgegengewirkt.

In diesem Zusammenhang erstrecken sich die drei Öffnungen entsprechend Anspruch 6 vorteilhafterweise jeweils etwa über ein Kreissegment von 60°.

In weiterer Ausgestaltung der Erfindung nach den Merkmalen der Ansprüche 7 und 8 weist das Verschlußelement, das im wesentlichen scheibenförmig ausgebildet ist, drei etwa gleich große Ausnehmungen auf, die über den Umfang des Verschlußelementes gleichmäßig verteilt sind, wobei sich die Bereiche des Verschlußelementes zwischen den Ausnehmungen jeweils etwa über ein Kreissegment von 60° erstrecken. Durch diese besondere Ausgestaltung des Verschlußelementes können die drei Öffnungen im stirnseitigen Bereich der Verankerungsschale vollständig ebenso geöffnet wie auch verschlossen werden.

Darüber hinaus liegt es gemäß Anspruch 9 im Rahmen der Erfindung, daß die Drehachse des Verschlußelementes mit der Längs- bzw. Rotationsachse der Verankerungsschale zusammenfällt, wodurch sich die konstruktive Ausgestaltung der erfindungsgemäßen Verankerungsschale erheblich vereinfacht.

Als besonders vorteilhaft hat sich dabei gemäß den Maßnahmen nach Anspruch 10 erwiesen, daß das Verschlußelement an dem stirnseitigen Bereich mit einer Niete oder dergleichen drehbar angebracht ist, die eine mit dem Gewinde versehene Bohrung aufweist, wodurch sich die konstruktive Ausgestaltung der erfindungsgemäßen Verankerungsschale noch weiter vereinfachen läßt.

Zudem liegt es im Rahmen der Erfindung entsprechend den Merkmalen nach Anspruch 11, das Verschlußelement an dem stirnseitigen Bereich ohne Spiel, insbesondere unter elastischer Vorspannung anzubringen. Hierdurch wird ein selbsttätiges Öffnen des Verschlußelementes in Folge einer späteren nicht ganz auszuschließenden Relativbewegung bzw. Reibung zwischen dem Verschlußelement einerseits und dem Pfannenkörper andererseits verhindert.

Um den Pfannenkörper in der Verankerungsschale nach deren Einbringen bzw. Eindrehen in die dafür vorgesehene Ausnehmung des Beckenknochens mittels einer Rast-, Schnapp- oder dergleichen -vorrichtung sicher zu fixieren, sind an der Innenfläche der einen konisch zulaufenden Wand wenigstens eine, vorzugsweise zwei bis vier umlaufende Ringsnuten oder dergleichen vorgesehen, die mit auf der Außenfläche des Pfannenkörpers entsprechend angeordneten Vorsprüngen zusammenwirken.

In weiterer Ausgestaltung der Erfindung sind der stirnseitige Bereich und die beiden konisch zulaufenden Wände nach Anspruch 12, wenigstens auf deren Außenfläche aufgerauht.

Vorzugsweise weisen der stirnseitige Bereich und die beiden konisch zulaufenden Wände eine durchschnittliche (Gauß'sche Verteilung) Oberflächenrauhigkeit von 5 µ auf. Auf diese Weise wird der Biomechanismus der Osteointegration besonders gefördert.

Das auf der Außenfläche der einen konisch zulaufenden Wand angeordnete, selbstschneidende Gewinde ist vorteilhafterweise durch eine vielzahl von Zähnen gebildet ist, deren Größe, Dicke und Höhe an die jeweilige Größe des Pfannenkörpers angepaßt ist.

Schließlich erfährt die erfindungsgemäße Verankerungsschale noch eine weitere Ausgestaltung dadurch, daß das auf der Außenfläche der einen konisch zulaufenden Wand angeordnete, selbstschneidende Gewinde durch eine Vielzahl von Zähnen gebildet ist, deren Form an die jeweilige Qualität des Beckenknochens, insbesondere an einen harten und sklerotischen bzw. einen weichen und porotischen Knochen, angepaßt ist. Demnach kann sowohl bei der Primär- als auch bei der Revisionsversorgung eine individuell an die Qualität des jeweiligen Knochenmaterials im Beckenbereich angepaßte Verankerungsschale von dem Operateur während des operativen Eingriffes ausgewählt werden. Die spezielle Form der Zähne trägt hauptsächlich auch bei besonders hartem Knochenmaterial dazu bei, daß die Kontaktfläche zwischen Knochen und Implantat wesentlich vergrößert wird und insofern die Verankerung nicht ausschließlich über die Spitzen der jeweiligen in das umliegende Knochenmaterial eingebrachten Zähne erfolgt. Hieraus ergibt sich der weitere Vorteil der Vermeidung punktueller Spannungsspitzen, die gewöhnlich zur Knochenresorption und somit zur Heilungsverzögerung, wenn nicht sogar zu Einheilungsstörungen führen können. Die insbesondere aus der Geometrie der einzelnen Zähne resultierende hohe Schneidfähigkeit des selbstschneidenden Gewindes verringert darüber hinaus nicht nur die Drehmomente beim Einbringen bzw. Eindrehen der Erfindungsgemäßen Verankerungsschale, sondern insgesamt auch die Qualität der Implantation.

Weitere Merkmale, Vorteile und Einzelheiten der Erfindung ergeben sich aus der nachfolgenden Beschreibung einer bevorzugten Ausführungsform der Erfindung sowie anhand der Zeichnung. Hierbei zeigen:
- Fig. 1: eine perspektivische Oberansicht einer Ausführungsform einer erfindungsgemäß ausgebildeten Verankerungsschale,
- Fig. 2: eine Oberansicht der Ausführungsform der erfindungsgemäß ausgebildeten Verankerungsschale nach Fig. 1,
- Fig. 3: eine Unteransicht der Ausführungsform der erfindungsgemäß ausgebildeten Verankerungsschale nach Fig. 1 und 2,
- Fig. 4: eine Seitenansicht der Ausführungsform der erfindungsgemäß ausgebildeten Verankerungsschale nach Fig. 1 bis 3, sowie
- Fig. 5: einen mittleren Halbschnitt durch die Ausführungsform der erfindungsgemäß ausgebildeten Verankerungsschale nach Fig. 1 bis 4 in vergrößertem Maßstab.

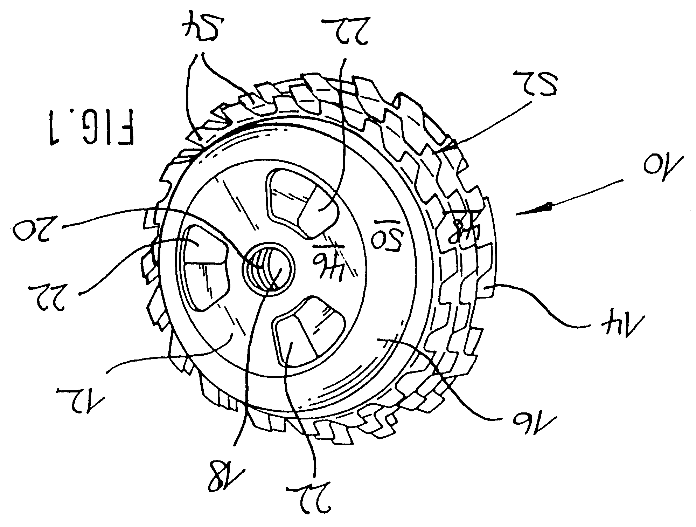

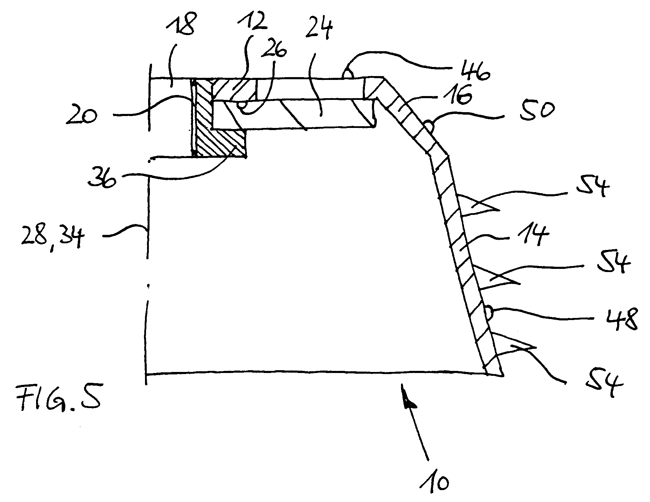

Die in Fig. 1 bis 5 dargestellte Verankerungsschale 10 ist zur Aufnahme eines nicht gezeigten Pfannenkörpers einer künstlichen, im Beckenknochen zementfrei verankerbaren Hüftgelenkspfanne, in die ein ebenfalls nicht gezeigter Gelenkkugelkopf einer Femurkopfprothese eingreifen soll, vorgesehen. Die Verankerungsschale 10 besteht aus Metall oder dergleichen Material, wie beispielsweise aus Titan oder einer Titanlegierung. Der Pfannenkörper selbst besteht hingegen aus Kunststoff oder dergleichen Material, z. B. aus Polyethylen etc.

Die Verankerungsschale 10 ist von einem dem Beckenknochen zugewandten stirnseitigen Bereich 12, einer sich an den stirnseitigen Bereich 12 anschließenden, konisch zulaufenden Wand 16 und einer weiteren konisch zulaufenden Wand 14, die sich an die erste Wand 16 anschließt, gebildet.

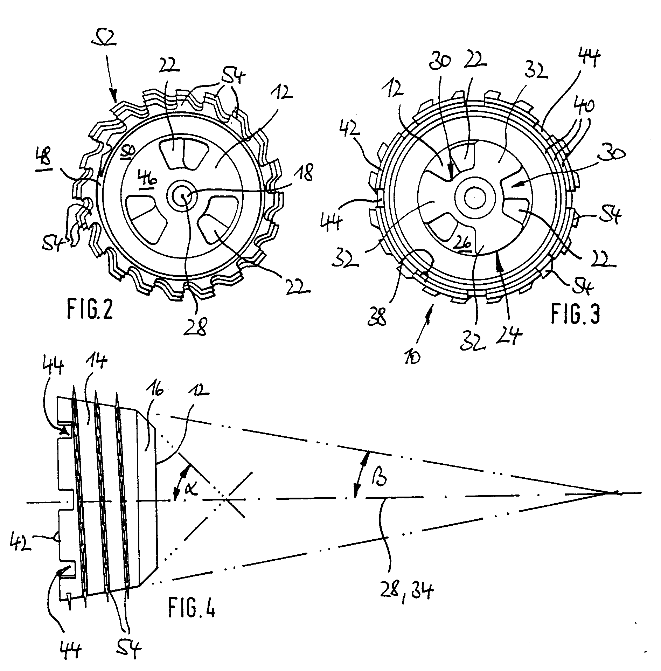

Entsprechend Fig. 4 ist der Konuswinkel α der konisch zulaufenden Wand 16 größer als der Konuswinkel β der einen konisch zulaufenden Wand 14. Vorzugsweise beträgt der Konuswinkel α der konisch zulaufenden Wand 16 etwa 30° bis 60°, insbesondere ca. 45°. Demgegenüber liegt der Konuswinkel β der weiteren konisch zulaufendnen Wand 14 etwa im Bereich zwischen 5° bis 20°, vorteilhafterweise bei etwa 10°.

Der stirnseitige Bereich 12 weist eine zentrale Bohrung 18 mit einem Gewinde 20 zur Aufnahme eines Einsetzinstrumentes (nicht gezeigt) auf.

Des weiteren ist im stirnseitigen Bereich 12 mindestens eine Öffnung 22 vorgesehen, die durch ein Verschlußelement 24 oder dergleichen verschließbar ist. Das Verschlußelement 24 liegt dabei auf der Innenfläche 26 des stirnseitigen Bereiches 12 auf und ist um eine senkrecht zum stirnseitigen Bereich 12 verlaufende Drehachse 28 verschwenkbar.

Bei dem Ausführungsbeispiel der erfindungsgemäßen Verankerungsschale 10 nach den Fig. 1 bis 5 sind drei etwa gleich große Öffnungen 22 vorgesehen, die über den Umfang des stirnseitigen Bereiches 12 gleichmäßig verteilt sind. Wie in Fig. 2 deutlich gezeigt, erstrecken sich die drei Öffnungen 22 dabei jeweils über ein Kreissegment von etwa 60°.

Das Verschlußelement 24 ist bei diesem Ausführungsbeispiel der Verankerungsschale 10 im wesentlichen scheibenförmig ausgebildet. Dabei weist das Verschlußelement 24 drei etwa gleich große Ausnehmungen 30 auf, die über den Umfang des Verschlußelementes 24 gleichmäßig verteilt sind. Die Bereiche 32 des Verschlußelementes 24 zwischen den Ausnehmungen 30 erstrecken sich jeweils über ein Kreissegment von ebenfalls etwa 60°. Insofern lassen sich sämtliche drei Öffnungen 22 in dem stirnseitigen Bereich 12 durch Verdrehen des scheibenförmigen Verschlußelementes 24 von den zwischen den Ausnehmungen 30 gebildeten Bereichen 32 des Verschlußelementes 24 gleichzeitig teilweise bzw. vollständig verschließen.

Die Drehachse 28 des Verschlußelementes 24 gemäß Fig. 1 bis 5 fällt mit der Längs- bzw. Rotationsachse 34 der Verankerungsschale 10 zusammen. Aus Gründen einer besonders einfachen, unaufwendigen Bauweise ist das Verschlußelement 24 an dem stirnseitigen Bereich 12 der Verankerungsschale 10 mit einer Niete 36 oder dergleichen drehbar angebracht, welche die mit dem Gewinde 20 versehene Bohrung 18 aufweist. Vorteilhafter Weise ist das Verschlußelement 24 an dem stirnseitigen Bereich 12 ohne Spiel und insbesondere unter elastischer Vorspannung drehbar befestigt.

Die Verbindung der erfindungsgemäßen Verankerungsschale 10 mit dem nicht dargestellten Pfannenkörper erfolgt mittels einer Rast-, Schnapp- oder dergleichen -vorrichtung. Zu diesem Zweck ist insbesondere an der Innenfläche 38 der weiteren konisch zulaufenden Wand 14 wenigstens eine umlaufende Ringnut 40 oder dergleichen vorgesehen, welche auf der Außenfläche des Pfannenkörpers entsprechend angeordnete, korrespondierende Vorsprünge aufnimmt. Die Innenfläche 38 der weiteren konisch zulaufenden Wand 14 ist entsprechend Fig. 3 mit insgesamt drei derartiger Ringnuten 40 versehen.

Am freien Rand 42 der weiteren konisch zulaufenden Wand 14 sind des weiteren eine Vielzahl von Ausnehmungen 44 gleichmäßig verteilt angeordnet, in die das jeweilige Einsetzinstrument beim Einbringen bzw. Eindrehen der Verankerungsschale 10 in die vorgefräste Ausnehmung des Beckenknochens während des operativen Eingriffs zur zusätzlichen Kraftübertragung eingreift. Darüber hinaus wirken die Ausnehmungen 44 mit korrespondierenden, zapfenartigen Vorsprüngen an dem Pfannenkörper zusammen, um den Pfannenkörper gleichzeitig gegen Verdrehung zu sichern.

Der stirnseitige Bereich 12 und die beiden konisch zulaufenden Wände 14, 16 sind weiterhin wenigstens auf deren Außenflächen 46, 48, 50 beispielsweise durch Sandstrahlung oder dergleichen aufgerauht. Insbesondere weisen die Außenfläche 46 des stirnseitigen Bereiches 12, die Außenfläche 48 der konisch zulaufenden Wand 14 und die Außenfläche 50 der konisch zulaufenden Wand 16 eine (durchschnittliche Gauß'sche Verteilung) Oberflächenrauhigkeit von 5 µm auf.

Gemäß den Fig. 1 bis 5 ist auf der Außenfläche 48 der weiteren konisch zulaufenden Wand 14 ein selbstschneidendes Gewinde 52 angeordnet. Das selbstschneidende Gewinde 52 wird durch eine Vielzahl von einzelnen, in Reihe hintereinander angeordneten und sägezahnartig ausgebildeten (Schneid-) Zähnen 54 gebildet. Dabei sind die Größe, Dicke und Höhe der Zähne 54 an die jeweilige Größe des Pfannenkörpers angepaßt. Darüber hinaus ist die Form bzw. geometrische Ausbildung der Zähne 54 entsprechend der jeweiligen Qualität des Beckenknochens, d. h. abhängig von einem harten und sklerotischen bzw. einem weichen und porotischen Knochenmaterial, vielseitig variierbar.

## Patentansprüche

1. Verankerungsschale aus Metall zur Aufnahme eines Pfannenkörpers aus Kunststoff einer künstlichen, im Beckenknochen zementfrei verankerbaren Hüftgelenkspfanne, wobei die Verankerungsschale (10) von einem dem Beckenknochen zugewandten, stirnseitigen Bereich (12) mit einer zentralen, mit einem Gewinde (20) versehenen Bohrung (18) zur Aufnahme eines Einsetzinstrumentes und mit mindestens einer Öffnung (22) sowie wenigstens einer sich an den stirnseitigen Bereich (12) anschließenden, konisch zulaufenden Wand (16) mit einem auf der Außenfläche (48) angeordneten, selbstschneidenden Gewinde (52) gebildet ist,
**dadurch gekennzeichnet,** daß
die mindestens eine Öffnung (22) im stirnseitigen Bereich (12) der Verankerungsschale (10) durch ein Verschlußelement (24) verschließbar ist, das auf der Innenfläche (26) des stirnseitigen Bereichs (12) aufliegt und um eine senkrecht zum stirnseitigen Bereich (12) verlaufende Drehachse (28) verschwenkbar ist.

2. Verankerungsschale nach Anspruch 1,
**dadurch gekennzeichnet,**
daß sich an die an den stirnseitigen Bereich (12) der Verankerungsschale (10) anschließende konisch zulaufende Wand (16) eine weitere konisch zulaufende Wand (14) schließt.

3. Verankerungsschale nach Anspruch 1 oder 2,
**dadurch gekennzeichnet**,
daß der Konuswinkel α der sich an den stirnseitigen Bereich (12) der Verankerungsschale (10) anschließenden konisch zulaufenden Wand (16) größer ist als der Konuswinkel β der weiteren konisch zulaufenden Wand (14).

4. Verankerungsschale nach Anspruch 3
**dadurch gekennzeichnet**,
daß der Konuswinkel α der sich an den stirnseitigen Bereich (12) der Verankerungsschale (10) anschließenden konisch zulaufenden Wand (16) etwa 30° bis 60°, insbesondere etwa 45°, beträgt.

5. Verankerungsschale nach einem der Ansprüche 1 bis 4,
**dadurch gekennzeichnet**,
daß drei etwa gleich große Öffnungen (22) vorgesehen sind, die über den Umfang des stirnseitigen Bereichs (12) gleichmäßig verteilt sind.

6. Verankerungsschale nach Anspruch 5,
**dadurch gekennzeichnet**,
daß sich die drei Öffnungen (22) jeweils über ein Kreissegment von etwa 60° erstrecken.

7. Verankerungsschale nach Anspruch 6,
**dadurch gekennzeichnet**,
daß das Verschlußelement (24) im wesentlichen scheibenförmig ausgebildet ist und drei etwa gleich große Ausnehmungen (30) aufweist, die über den Umfang des Verschlußelementes (24) gleichmäßig verteilt sind.

8. Verankerungsschale nach Anspruch 7,
**dadurch gekennzeichnet**,
daß sich die Bereiche (32) des Verschlußelementes (24) zwischen den Ausnehmungen (30) jeweils über ein Kreissegment von etwa 60° erstrecken.

9. Verankerungsschale nach einem der Ansprüche 1 bis 8,
**dadurch gekennzeichnet**,
daß die Drehachse (28) des Verschlußelementes (24) mit der Längs- bzw. Rotationsachse (34) der Verankerungsschale (10) zusammenfällt.

10. Verankerungsschale nach Anspruch 9,
**dadurch gekennzeichnet**,
daß das Verschlußelement (24) an dem stirnseitigen Bereich (12) mit einer Niete (36) oder dergleichen drehbar angebracht ist, die die mit dem Gewinde (20) versehene Bohrung (18) aufweist.

11. Verankerungsschale nach einem der Ansprüche 1 bis 10,
**dadurch gekennzeichnet**,
daß das Verschlußelement (24) an dem stirnseitigen Bereich (12) ohne Spiel, insbesondere unter elastischer Vorspannung angebracht ist.

12. Verankerungsschale nach einem der Ansprüche 1 bis 11,
**dadurch gekennzeichnet**,
daß der stirnseitige Bereich (12) und die beiden konisch zulaufenden Wände (14, 16) wenigstens auf deren Außenflächen (46, 48, 50) aufgerauht sind, insbesondere eine Aufrauhung nach Gauß'scher Verteilung von 5 µm aufweisen.

## Claims

1. Fixation shell made of metal for receiving a cup body made of plastic of an artificial acetabulum which can be fixed in the pelvic bone without cement, the fixation shell (10) being formed by an end region (12) facing the pelvic bone and having a central bore (18), which is provided with a screw-thread (20) and is intended for receiving an insertion instrument, and having at least one opening (22) and at least one conically tapering wall (16) which adjoins the end region (12) and has a self-tapping screw-thread (52) arranged on the outer surface (48), characterized in that the at least one opening (22) in the end region (12) of the fixation shell (10) can be closed by a closure element (24) which rests on the inner surface (26) of the end region (12) and can be pivoted about an axis of rotation (28) running perpendicular to the end region (12).

2. Fixation shell according to Claim 1, characterized in that a further conically tapering wall (14) adjoins the conically tapering wall (16) which adjoins the end region (12) of the fixation shell (10).

3. Fixation shell according to Claim 1 or 2, characterized in that the cone angle α of the conically tapering wall (16) which adjoins the end region (12) of the fixation shell (10) is larger than the cone angle β of the further conically tapering wall (14).

4. Fixation shell according to Claim 3, characterized in that the cone angle α of the conically tapering wall (16) which adjoins the end region (12) of the fixation shell (10) is about 30° to 60°, in particular about 45°.

5. Fixation shell according to one of Claims 1 to 4, characterized in that three openings (22) of approximately equal size are provided, which are distributed evenly over the periphery of the end region (12).

6. Fixation shell according to Claim 5, characterized in that the three openings (22) each extend over a segment of a circle of about 60°.

7. Fixation shell according to Claim 6, characterized in that the closure element (24) is of essentially disc-shaped design and has three cutouts (30) of approximately equal size which are distributed evenly over the periphery of the closure element (24).

8. Fixation shell according to Claim 7, characterized in that the regions (32) of the closure element (24) between the cutouts (30) each extend over a segment of a circle of about 60°.

9. Fixation shell according to one of Claims 1 to 8, characterized in that the axis of rotation (28) of the closure element (24) coincides with the longitudinal axis or axis of rotation (34) of the fixation shell (10).

10. Fixation shell according to Claim 9, characterized in that the closure element (24) is rotatably mounted on the end region (12) by means of a rivet (36) or the like, which has the bore (18) provided with the screw-thread (20).

11. Fixation shell according to one of Claims 1 to 10, characterized in that the closure element (24) is mounted on the end region (12) without play, in particular with elastic prestress.

12. Fixation shell according to one of Claims 1 to 11, characterized in that the end region (12) and the two conically tapering walls (14, 16) are roughened at least on their outer surfaces (46, 48, 50), in particular they have a roughening of 5 µm according to a normal distribution.

## Revendications

1. Coquille de fixation en métal pour recevoir la coupole en plastique d'une coupole de hanche synthétique prévue pour être ancrée sans cément dans l'os du bassin, cette coquille de fixation (10) étant composée d'une partie frontale (12) orientée vers l'os du bassin présentant un perçage central (18) prévu avec un taraudage (20) pour recevoir un instrument d'installation et prévu avec au moins une ouverture (22), ainsi qu'au moins une paroi (16) se reliant à la partie frontale (12), s'étendant de manière conique et étant équipée d'un filetage (52) autotaraudant en sa face externe (48), caractérisée en ce qu'au moins une ouverture (22) en la partie frontale (12) de la coquille de fixation (10) peut être fermée par un élément de fermeture (24) reposant en la face interne (26) de la partie frontale (12) et mobile en rotation autour d'un axe (28) perpendiculaire à la partie frontale (12).

2. Coquille de fixation selon la revendication 1, caractérisée en ce qu'une autre paroi (14) s'étendant de manière conique est reliée à la paroi (16) s'étendant de manière conique elle-même reliée en la partie frontale (12) de la coquille de fixation (10).

3. Coquille de fixation selon la revendication 1 ou 2, caractérisée en ce que l'angle au sommet α de la paroi (16) s'étendant de manière conique et se fermant en la partie frontale (12) de la coquille de fixation (10) est plus grand que l'angle au sommet β de l'autre paroi (14) s'étendant de manière conique.

4. Coquille de fixation selon la revendication 3, caractérisée en ce que l'angle au sommet α de la paroi (16) s'étendant de manière conique et se fermant en la partie frontale (12) de la coquille de fixation (10) est compris entre 30 et 60°, en particulier de l'ordre de 45°.

5. Coquille de fixation selon l'une des revendications 1 à 4, caractérisée en ce qu'il est prévu trois ouvertures (22) de grandeur sensiblement identique et réparties à intervalles réguliers sur la périphérie de la partie frontale (12).

6. Coquille de fixation selon la revendication 5, caractérisée en ce que les trois ouvertures (22) s'étendent chacune sur un secteur circulaire d'environ 60°.

7. Coquille de fixation selon la revendication 6, caractérisée en ce que l'élément de fermeture (24) est réalisé sous la forme sensiblement d'un disque présentant trois ouvertures (30) de grandeur sensiblement identique réparties à intervalles réguliers sur la périphérie de l'élément de fermeture (24).

8. Coquille de fixation selon la revendication 7, caractérisée en ce que les zones (32) de l'élément de fermeture (24) situées entre les ouvertures (30) s'étendent respectivement sur un secteur circulaire d'environ 60°.

9. Coquille de fixation selon l'une des revendications 1 à 8, caractérisée en ce que l'axe de rotation (28) de l'élément de fermeture (24) coïncide avec l'axe longitudinal, respectivement de rotation, (34) de la coquille de fixation (10).

10. Coquille de fixation selon la revendication 9, caractérisée en ce que l'élément de fermeture (24) est rapporté mobile en rotation en la partie frontale (12) par un rivet (36) ou analogue présentant le perçage (18) prévu avec un taraudage (20).

11. Coquille de fixation selon l'une des revendications 1 à 10, caractérisée en ce que l'élément de fermeture (24) est rapporté sans jeu, en particulier sous tension élastique, en la partie frontale (12).

12. Coquille de fixation selon l'une des revendications 1 à 11, caractérisée en ce que la partie frontale (12) et les deux parois s'étendant de manière conique (14,16) sont granuleuses au moins en leurs faces externes (46,48,50), en présentant une granulométrie de 5 µm selon la répartition de Gauss.
